# EUROPEAN PATENT SPECIFICATION

(11) **EP 3 248 557 B1**
(45) Date of publication and mention of the grant of the patent: **08.01.2020**
(21) Application number: 17173060.9
(22) Date of filing: 26.05.2017
(51) Int. Cl.: A61B 17/70, A61B 17/86

(54) **SPINAL SCREW FIXATION DEVICE**
SPINALE FIXATIONSVORRICHTUNG MIT SCHRAUBEN
DISPOSITIF DE FIXATION VERTEBRAL A VIS

(30) Priority: 26.05.2016 US 201662341704 P
(43) Date of publication of application: 29.11.2017
(73) Proprietor: Wu, Meng-Huang, Taipei city 110 (TW)
(72) Inventor: WU, Meng-Huang, 110 Taipei City (TW); TSAI, Pei-I, 300 Hsinchu City (TW); YANG, Kuo-Yi, 300 Hsinchu City (TW); HUANG, Chih-Chieh, 350 Zhunan Township, Miaoli County (TW); LIN, Shih-Ping, 812 Kaohsiung (TW); WEN, Yi-Hung, 300 Hsinchu City (TW); FAN, Wei-Luan, 350 Zhunan Township, Miaoli County (TW)
(74) Representative: Krauns, Christian

(56) References cited:
- WO-A2-2009/044395
- US-A- 5 425 767
- US-A1- 2006 030 852
- US-A1- 2013 053 901
- US-A1- 2016 128 732

## Description

### BACKGROUND OF THE INVENTION

### Technical Field

The invention relates in general to a fixation device, and more particularly to a spinal screw fixation device.

### Description of the Related Art

In clinical practice, one or multiple bone screws are commonly used in the spinal fracture surgery, the spinal corrective surgery, the non-fusion spinal surgery and the spinal fusion surgery. The spinal fixation device using pedicle screws has an excellent fixation effect and is widely used. However, the pedicle screw must be inserted into the spine from the outside, and therefore needs a larger opening for the wound. Since the cortical bone located on the outer layer of the bone is hard, the spinal fixation device using cortical screws provides better fixation effect than the spinal fixation device using pedicle screws. Furthermore, the cortical screw is inserted into the spine from the inside, so the opening for the wound is smaller. However, since the harder part of the cortical bone often requires the use of an bone driller, the cortical screw is not widely used in clinical practice.

In recent years, when an osteoporosis patient receives the spinal surgery, it often happens that the pedicle screw becomes loose and makes the surgery failed. Therefore, how to enhance the fixation effect of the bone screw and reduce the probability of surgery failure has become a prominent task for the industry.

The WO 2009/044395 A2 discloses a self-locking fastening device comprising fastening elements. The fastening elements are adapted to be inserted into a bone. The fastening elements are provided with a bore at a portion thereof to be inserted into the bone. The fastening elements are adapted for consecutively interlocking in a body of said bone by means of inserting the fastening elements into the bone so that each successive element passes through said bore of each preceding element. The US 2006/0030852 A1 discloses an anchoring system for mounting objects to a bone, comprising first and second anchoring members each having proximal and distal ends. The proximal ends are adapted to hold the object to the bone and are spaced from each other with the first and second anchoring members converging from the proximal ends towards the distal ends. The anchoring members are adapted to be connected to each other in the bone and distally of the proximal ends. There are provided at least two first anchoring members each adapted to be connected to the second anchoring member. The second anchoring member and each of the first anchoring members define co-operating engagement means for connecting each first anchoring member to the second anchoring member. The US 5,425,767 A discloses an anchor for an artificial ligament in a bone. The anchor comprises a Y-shaped socket having first and second arms configured at an acute angle with respect to each other. The first arm is sized to receive the ligament and has cross-grooves for gripping the ligament. A bullet shaped clamp element is rotatably mounted to the second arm. The clamp element also has grooves that directly contact the ligament to apply an oblique force against the ligament. The clamp element and the cross-grooves in the first arm completely surround and compress the ligament to frictionally secure the ligament to the socket. The US 2016/0128732 A1 discloses devices, systems and methods for surgical fixation, including multi-screw anchoring devices for anchoring to various anatomical locations such as a sacral level or other anatomy of the spine. The anchoring devices include screw assemblies that are adjustable in a variety of different ways. The US 2013/0053901 A1 discloses a cannulated bone anchor assembly including a bone anchor, a receiver member for receiving a spinal fixation element to be coupled to the bone anchor, and a closure mechanism to fix the spinal fixation element with respect to the receiver member. The bone anchor includes a distal shaft having a first threaded section proximal a proximal head, a second threaded section proximal to and adjacent the first threaded section, and a third threaded section proximal to and adjacent the second threaded section. The first threaded section has a constant major and minor diameter. The second threaded section has a tapering major and minor diameter. The third threaded section has a tapering major diameter and a constant minor diameter.

### SUMMARY OF THE INVENTION

The present invention is directed to a spinal screw fixation device, which provides a 3D locking effect through a three-point fixed structure constituted by a spinal screw, a fixing screw and a locking plate to reduce the risk of the spinal screw becoming loose and pulled out.

According to one embodiment of the present invention, a spinal screw fixation device is provided. The spinal screw fixation device includes a spinal screw, a fixing screw, and a locking plate. The spinal screw has an oblique perforation hole through which the fixing screw passes. The locking plate has two locking holes through which the spinal screw and the fixing screw pass, respectively. The spinal screw, the fixing screw and the locking plate constitute a three-point fixed structure.

The above and other aspects of the invention will become better understood with regard to the following detailed description of the preferred but non-limiting embodiment(s). The following description is made with reference to the accompanying drawings.

### BRIEF DESCRIPTION OF THE DRAWINGS

FIGS. 1A and 1B respectively are a schematic diagram and an explosion diagram of a spinal screw fixation device according to an embodiment of the invention.
FIG. 2 is a schematic diagram of a direction in which the spinal screw fixation device is inserted into a spine.
FIG. 3 is a schematic diagram of a spinal screw fixation device being inserted into a spine.
FIG. 4 is a schematic diagram of a U-shaped base and a rotatable thread body of a spinal screw.
FIG. 5 is a cross-sectional view of a hollowed portion of a spinal screw.
FIG. 6 is a schematic diagram of a drill guide plate for guiding the drilling direction.

### DETAILED DESCRIPTION OF THE INVENTION

Detailed descriptions of the invention are disclosed below with a number of embodiments. However, the disclosed embodiments are for explanatory and exemplary purposes only, not for limiting the scope of protection of the invention. Designations common to the accompanying drawings are used to indicate identical or similar elements.

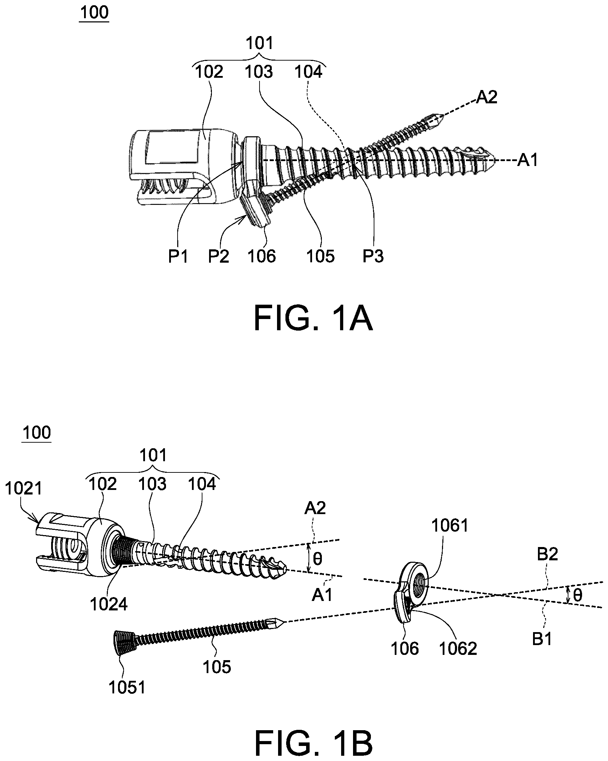

Refer to FIGS. 1A, 1B, 2 and 3. The spinal screw fixation device 100 according to an embodiment of the invention includes a spinal screw 101, a fixing screw 105 and a locking plate 106. The spinal screw 101 has a top 102 and a thread body 103. The top 102 includes a U-shaped base 1021 for receiving a vertebral fixing rod (not illustrated). The surface of the thread body 103 has an outer thread through which the thread body 103 can be screwed into the spine 200. The spinal screw 101 has an oblique perforation hole 104 through which the thread body 103 passes. The exit direction of the oblique perforation hole 104 is different from the axial direction of the thread body 103. In an embodiment, the exit direction of the oblique perforation hole 104 (that is, the inserting direction A2) and the axial direction of the thread body 103 (that is, the inserting direction A1) form an angle θ, such as between 15-20 degrees or between 15-35 degrees. The fixing screw 105 passes through the spinal screw 101 via the oblique perforation hole 104, such that the spinal screw 101 and the fixing screw 105 intersect in the oblique perforation hole 104.

Besides, the locking plate 106 has two locking holes, that is, the first locking hole 1061 and the second locking hole 1062. The exit direction B1 of the first locking hole 1061 is different from the exit direction B2 of the second locking hole 1062. The exit direction B1 of the first locking hole 1061 is aligned with the axial direction of the thread body 103 (that is, the inserting direction A1). The exit direction B2 of the second locking hole 1062 is aligned with the exit direction of oblique perforation hole 104 (that is, the inserting direction A2). Thus, when the thread body 103 of the spinal screw 101 passes through the first locking hole 1061, the thread body 103 can be positioned by the first locking hole 1061, such that the thread body 103 can be screwed into the spine 200 according to a screw-in path to avoid the spinal screw 101 being skewed or misplaced. On the other hand, when the fixing screw 105 passes through the second locking hole 1062, the fixing screw 105 can be positioned by the second locking hole 1062, such that the fixing screw 105, after passing through the oblique perforation hole 104, can enter the spine according to a predetermined path to assist with the positioning of the spinal screw 101. The first locking hole 1061 and the second locking hole 1062 are two screw holes, for example.

As indicated in FIG. 1A, every two of the spinal screw 101, the fixing screw 105 and the locking plate 106 are engaged with each other to constitute a three-point fixed structure. The first end (that is, the engaging portion 1024) of the top 102 of the spinal screw 101 is received in the first locking hole 1061 to form a first fixing point P1. A part (that is, the engaging portion 1051) of the fixing screw 105 is received in the second locking hole 1062 to form a second fixing point P2. The spinal screw 101 and the fixing screw 105 are engaged with each other in the oblique perforation hole 104 to form a third fixing point P3. Thus, a 3D locking effect is generated by the three fixing points.

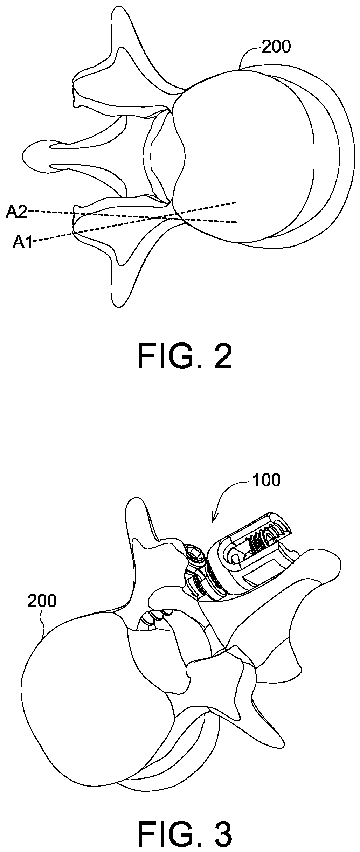

In the present embodiment, the spinal screw 101 is such as a pedicle screw or a cortical screw. The pedicle screw and the cortical screw are inserted into the spine 200 in different directions, that is, the pedicle screw is inserted into the spine 200 from outside (the inserting direction A1 as indicated in FIG. 2), and the cortical screw is inserted into the spine 200 from inside (the inserting direction A2 as indicated in FIG. 2). In an embodiment, when the pedicle screw is used as a main spinal screw 101 for fixation purpose, the pedicle screw is firstly inserted into the spine 200 along the inserting direction A1. Then, a cortical screw, a fixing rod or a fixing piece is used as an auxiliary fixing screw 105. The fixing screw 105 passes through the pedicle screw to be inserted into the cortical bone on the outer layer of the bone along the inserting direction A2 to enhance the 3D locking effect. In another embodiment, when the cortical screw is used as a main spinal screw 101 for fixation purpose, the cortical screw is firstly inserted into the spine 200 along the inserting direction A2. Then, a pedicle screw, a fixing rod or a fixing piece is used as an auxiliary fixing screw 105. The fixing screw 105 passes through the cortical screw to be inserted into the pedicle of spine along the inserting direction A1 to enhance the 3D locking effect.

Reference(s) to "embodiment(s)" throughout the description which are not under the scope of the appended claims merely represent possible exemplary executions and are therefore not part of the present invention.

In an embodiment, when the pedicle screw is used as the spinal screw 101, the thread body 103 of the pedicle screw has a length between 30-110 mm, and a pitch circle diameter between 4.5-8.5 mm, but the invention is not limited thereto. When the cortical screw is used as the spinal screw 101, the thread body 103 of the cortical screw has a length between 30-110 millimeters (mm) and a pitch circle diameter between 4.5-8.5 mm, but the invention is not limited thereto. Besides, the fixing screw 105 has a length between 20-50 mm and a diameter between 1.5-3.5 mm but smaller than the diameter of the spinal screw 101. Also, the inner circle diameter of the oblique perforation hole 104 of the spinal screw 101 is basically equivalent to the outer circle diameter of the fixing screw 105, such that the spinal screw 101 and the fixing screw 105 can be tightly engaged with each other. The oblique perforation hole 104 of the spinal screw 101 is not limited to a cylindrical hole, and a long column hole would also do. When the oblique perforation hole 104 is a cylindrical hole, the thread body 103 can form an engaging portion, such as a latch, on the inner surface of the oblique perforation hole 104. Thus, when the fixing screw 105 passes through the oblique perforation hole 104, the fixing screw 105 and the engaging portion are engaged with each other. When the oblique perforation hole 104 is a long column hole or has a non-cylindrical shape, the fixing screw 105, having a long column shape, can be engaged with the oblique perforation hole 104 to enhance the locking effect.

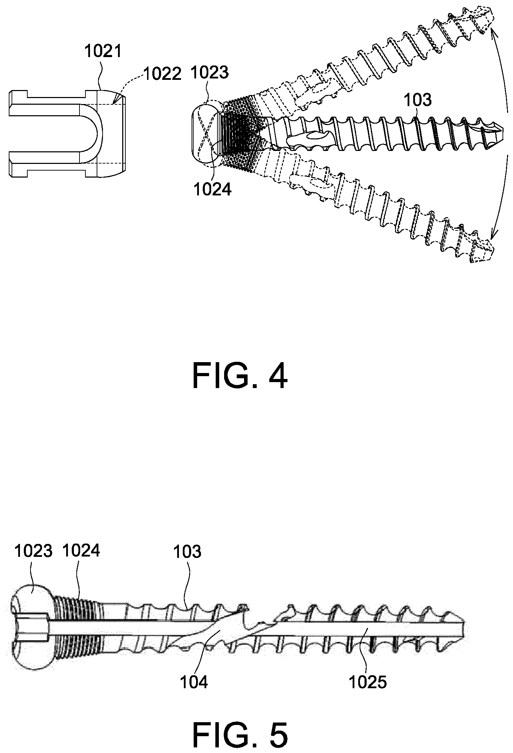

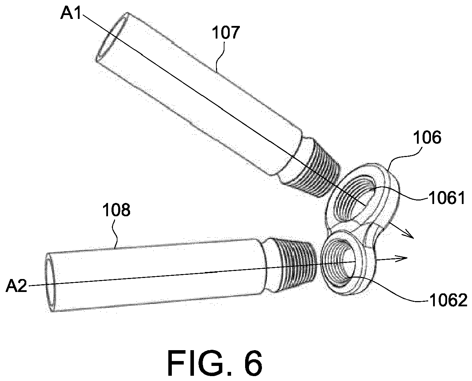

Refer to FIG. 4. In an embodiment, the top 102 includes a U-shaped base 1021 and an active joint 1023, wherein the active joint 1023 is received in an opening 1022 of the U-shaped base 1021, and the thread body 103 is rotatably connected to the U-shaped base 1021 via the active joint 1023, such that the thread body 103 has a steering function with respect to the U-shaped base 1021 and the vertebral fixing rod can be conveniently positioned in the U-shaped base 1021. Refer to FIG. 5. The thread body 103 is extended from the first end 1024 of the top 102, and the spinal screw 101 has a hollowed portion 1025. The hollowed portion 1025, passing through the thread body 103 from the active joint 1023 of the top 102, is used as a guide entrance in the micro-wound surgery. The hollowed portion 1025 has a diameter between 2-2.4 mm. Refer to FIG. 6. In the present embodiment, the first locking hole 1061 and the second locking hole 1062 of the locking plate 106 can further be used as the guide holes for the electrical driller. The first locking hole 1061 and the second locking hole 1062 combined with the guide pipes 107 and 108 can guide the drilling direction and reduce the risk of misplacing the pedicle screw and cortical screw.

The spinal screw fixation device disclosed in above embodiments of the invention generates a 3D locking effect through a three-point fixed structure constituted by a spinal screw, a fixing screw and a locking plate to reduce of the risk of the spinal screw becoming loose, reduce the probability of surgery failure and enhance patients' recovery from the spinal surgery.

## Claims

1. A spinal screw fixation device (100), comprising:
a spinal screw (101) having an oblique perforation hole (104);
a fixing screw (105) passing through the oblique perforation hole (104); and
a locking plate (106) having two locking holes (1061, 1062) through which the spinal screw (101) and the fixing screw (105) pass, wherein the spinal screw (101), the fixing screw (105) and the locking plate (106) constitute a three-point fixed structure, wherein one of the spinal screw (101) and the fixing screw (105) is a pedicle screw, and the other of the spinal screw (101) and the fixing screw (105) is a cortical screw.

2. The spinal screw fixation device (100) according to claim 1, wherein the spinal screw (101) is the pedicle screw, and the pedicle screw comprises a top (102) and a thread body (103), the thread body (103) is extended from a first end (1024) of the top (102), and the first end (1024) is received in one of the two locking holes (1061, 1062) to form a first fixing point,
the fixing screw (105) is a cortical screw, wherein a part of the fixing screw (105) is received in the other of the two locking holes (1061, 1062) to form a second fixing point, and the spinal screw (101) and the fixing screw (105) are engaged with each other in the oblique perforation hole (104) to form a third fixing point.

3. The spinal screw fixation device (100) according to claim 1, wherein the spinal screw (101) is the cortical screw, and the cortical screw comprises a top (102) and a thread body (103), the thread body (103) is extended from a first end (1024) of the top (102), and the first end (1024) is received in one of the two locking holes (1061, 1062) to form a first fixing point,
the fixing screw (105) is the pedicle screw, wherein a part of the fixing screw (105) is received in the other of the two locking holes (1061, 1062) to form a second fixing point, and the spinal screw (101) and the fixing screw (105) are engaged with each other in the oblique perforation hole (104) to form a third fixing point.

4. The spinal screw fixation device (100) according to claim 2 or 3, wherein the spinal screw (101) and the fixing screw (105) form an angle of 15 to 20 degrees and intersect in the oblique perforation hole (104).

5. The spinal screw fixation device (100) according to claim 2 or 3, wherein the top of the spinal screw (101) comprises a U-shaped base (1021) and an active joint (1023), the active joint (1023) is received in an opening (1022) of the U-shaped base (1021), and the thread body (103) is rotatably connected to the U-shaped base (1021) via the active joint (1023).

6. The spinal screw fixation device (100) according to claim 2 or 3, wherein the spinal screw (101) has a hollowed portion (1025) passing the thread body (103) from the top (102).

7. The spinal screw fixation device (100) according to claim 2 or 3, wherein the thread body (103) has an engaging portion on an inner surface of the oblique perforation hole (104), and the fixing screw (105) and the engaging portion are engaged with each other in the oblique perforation hole (104).

8. The spinal screw fixation device (100) according to claim 2 or 3, wherein the oblique perforation hole (104) and the fixing screw (105) match each other through a long column shape or a non-cylindrical shape.

## Patentansprüche

1. Wirbelsäulenschrauben-Fixierungsvorrichtung (100), welche aufweist:
eine Wirbelsäulenschraube (101), die ein schräges Perforationsloch (104) aufweist;
eine Befestigungsschraube (105), die durch das schräge Perforationsloch (104) verläuft; und
eine Verriegelungsplatte (106), die zwei Verriegelungslöcher (1061, 1062) aufweist, durch die die Wirbelsäulenschraube (101) und die Befestigungsschraube (105) verlaufen, wobei die Wirbelsäulenschraube (101), die Befestigungsschraube (105) und die Verriegelungsplatte (106) eine feste Struktur mit drei Punkten bilden, wobei der eine von der Wirbelsäulenschraube (101) und der Fixierschraube (105) eine Pedikelschraube ist, und die andere von der Wirbelsäulenschraube (101) und der Fixierschraube (105) eine Kortikalisschraube ist.

2. Wirbelsäulenschrauben-Fixierungsvorrichtung (100) nach Anspruch 1, wobei die Wirbelsäulenschraube (101) die Pedikelschraube ist und die Pedikelschraube eine Oberseite (102) und einen Gewindekörper (103) aufweist, wobei der Gewindekörper (103) sich von einem ersten Ende (1024) der Oberseite (102) erstreckt und das erste Ende (1024) in einem der beiden Verriegelungslöcher (1061, 1062) aufgenommen ist, um einen ersten Befestigungspunkt zu bilden,
die Befestigungsschraube (105) eine Kortikalisschraube ist, wobei ein Teil der Befestigungsschraube (105) in dem anderen der beiden Verriegelungslöcher (1061, 1062) aufgenommen ist, um einen zweiten Befestigungspunkt zu bilden, und die Wirbelsäulenschraube (101) und die Befestigungsschraube (105) in dem schrägen Perforationsloch (104) miteinander in Eingriff stehen, um einen dritten Befestigungspunkt zu bilden.

3. Wirbelsäulenschrauben-Fixiervorrichtung (100) nach Anspruch 1, wobei die Wirbelsäulenschraube (101) die Kortikalisschraube ist und die Kortikalisschraube eine Oberseite (102) und einen Gewindekörper (103) aufweist, wobei der Gewindekörper (103)) sich von einem ersten Ende (1024) der Oberseite (102) erstreckt und das erste Ende (1024) in einem der beiden Verriegelungslöcher (1061, 1062) aufgenommen ist, um einen ersten Befestigungspunkt zu bilden,
die Befestigungsschraube (105) die Pedikelschraube ist, wobei ein Teil der Befestigungsschraube (105) in dem anderen der beiden Verriegelungslöcher (1061, 1062) aufgenommen ist, um einen zweiten Befestigungspunkt zu bilden, und die Wirbelsäulenschraube (101) und die Befestigungsschraube (105) in dem schrägen Perforationsloch (104) miteinander in Eingriff stehen, um einen dritten Befestigungspunkt zu bilden.

4. Wirbelsäulenschrauben-Fixiervorrichtung (100) nach Anspruch 2 oder 3, wobei die Wirbelsäulenschraube (101) und die Fixierschraube (105) einen Winkel von 15 bis 20 Grad bilden und sich in dem schrägen Perforationsloch (104) schneiden.

5. Wirbelsäulenschrauben-Fixierungsvorrichtung (100) nach Anspruch 2 oder 3, wobei die Oberseite der Wirbelsäulenschraube (101) eine U-förmige Basis (1021) und ein Wirkgelenk (1023) aufweist, wobei das Wirkgelenk (1023) in einer Öffnung (1022) der U-förmigen Basis (1021) aufgenommen ist, und der Gewindekörper (103) drehbar mit der U-förmigen Basis (1021) über das Wirkgelenk (1023) verbunden ist.

6. Wirbelsäulenschrauben-Fixierungsvorrichtung (100) nach Anspruch 2 oder 3, wobei die Wirbelsäulenschraube (101) einen hohlen Abschnitt (1025) aufweist, der den Gewindekörper (103) von der Oberseite (102) durchläuft.

7. Wirbelsäulenschrauben-Fixiervorrichtung (100) nach Anspruch 2 oder 3, wobei der Gewindekörper (103) einen Eingriffsabschnitt an einer inneren Oberfläche des schrägen Perforationslochs (104) aufweist und wobei die Fixierschraube (105) und der Eingriffsabschnitt in dem schrägen Perforationsloch (104) miteinander in Eingriff stehen.

8. Wirbelsäulenschrauben-Fixierungsvorrichtung (100) nach Anspruch 2 oder 3, wobei das schräge Perforationsloch (104) und die Fixierungsschraube (105) durch eine lange Säulenform oder eine nicht-zylindrische Form zueinander passen.

## Revendications

1. Dispositif de fixation à vis spinale (100), comprenant :
une vis spinale (101) ayant un trou de perforation oblique (104) ;
une vis de fixation (105) passant à travers le trou de perforation oblique (104) ; et
une plaque de blocage (106) ayant deux trous de blocage (1061, 1062) à travers lesquels passent la vis spinale (101) et la vis de fixation (105), dans lequel la vis spinale (101), la vis de fixation (105) et la plaque de blocage (106) constituent une structure fixe à trois points, dans lequel l'une de la vis spinale (101) et de la vis de fixation (105) est une vis pédiculaire, et l'autre de la vis spinale (101) et de la vis de fixation (105) est une vis corticale.

2. Dispositif de fixation à vis spinale (100) selon la revendication 1, dans lequel la vis spinale (101) est la vis pédiculaire, et la vis pédiculaire comprend une partie haute (102) et un corps de filet (103), le corps de filet (103) est étendu depuis une première extrémité (1024) de la partie haute (102), et la première extrémité (1024) est reçue dans l'un des deux trous de blocage (1061, 1062) pour former un premier point de fixation,
la vis de fixation (105) est une vis corticale, dans lequel une partie de la vis de fixation (105) est reçue dans l'autre des deux trous de blocage (1061, 1062) pour former un deuxième point de fixation, et la vis spinale (101) et la vis de fixation (105) sont engagées l'une avec l'autre dans le trou de perforation oblique (104) pour former un troisième point de fixation.

3. Dispositif de fixation à vis spinale (100) selon la revendication 1, dans lequel la vis spinale (101) est la vis corticale, et la vis corticale comprend une partie haute (102) et un corps de filet (103), le corps de filet (103) est étendu depuis une première extrémité (1024) de la partie haute (102), et la première extrémité (1024) est reçue dans l'un des deux trous de blocage (1061, 1062) pour former un premier point de fixation,
la vis de fixation (105) est la vis pédiculaire, dans lequel une partie de la vis de fixation (105) est reçue dans l'autre des deux trous de blocage (1061, 1062) pour former un deuxième point de fixation, et la vis spinale (101) et la vis de fixation (105) sont engagées l'une avec l'autre dans le trou de perforation oblique (104) pour former un troisième point de fixation.

4. Dispositif de fixation à vis spinale (100) selon la revendication 2 ou 3, dans lequel la vis spinale (101) et la vis de fixation (105) forment un angle de 15 à 20 degrés et se coupent dans le trou de perforation oblique (104).

5. Dispositif de fixation à vis spinale (100) selon la revendication 2 ou 3, dans lequel la partie haute de la vis spinale (101) comprend une base en forme de U (1021) et un joint actif (1023), le joint actif (1023) est reçu dans une ouverture (1022) de la base en forme de U (1021), et le corps de filet (103) est raccordé en rotation à la base en forme de U (1021) via le joint actif (1023).

6. Dispositif de fixation à vis spinale (100) selon la revendication 2 ou 3, dans lequel la vis spinale (101) a une portion creusée (1025) passant dans le corps de filet (103) depuis la partie haute (102).

7. Dispositif de fixation à vis spinale (100) selon la revendication 2 ou 3, dans lequel le corps de filet (103) a une portion d'engagement sur une surface interne du trou de perforation oblique (104), et la vis de fixation (105) et la portion d'engagement sont engagées l'une avec l'autre dans le trou de perforation oblique (104).

8. Dispositif de fixation à vis spinale (100) selon la revendication 2 ou 3, dans lequel le trou de perforation oblique (104) et la vis de fixation (105) concordent l'un avec l'autre par une forme de colonne longue ou une forme non cylindrique.
